# EUROPEAN PATENT APPLICATION

(11) **EP 2 218 714 A1**
(43) Date of publication of application: **18.08.2010**
(21) Application number: 08848235.1
(22) Date of filing: 04.11.2008
(51) Int. Cl.: C07D 319/12, C08G 63/08, C08L 101/16

(54) **APPARATUS AND METHOD BOTH RELATING TO POLYMER SYNTHESIS**

(30) Priority: 07.11.2007 JP 2007289664
(71) Applicant: Hitachi Plant Technologies, Ltd., Toshima-ku Tokyo 170-8466 (JP); Toyota Jidosha Kabushiki Kaisha, Toyota-shi, Aichi 471-8571 (JP)
(72) Inventor: KAMIKAWA, Masayuki, Hitachi-shi Ibaraki 319-1221 (JP); MATSUO, Toshiaki, Hitachi-shi Ibaraki 319-1221 (JP); OKAMOTO, Naruyasu, Tokyo 170-8466 (JP); OKA, Kenichiro, Hitachi-shi Ibaraki 319-1221 (JP); YATSUGI, Takashi, Toyota-shi Aichi 471-8571 (JP); FUJII, Yasuhiro, Toyota-shi Aichi 471-8571 (JP); KAWAMOTO, Tatsushi, Toyota-shi Aichi 471-8571 (JP)
(74) Representative: Strehl Schübel-Hopf & Partner
(86) International application number: PCT/JP2008/070047
(87) International publication number: WO 2009/060832

(57) **Abstract**

The present invention provides a method and apparatus for producing polylactic acid by depolymerizing lactic acid oligomers and subjecting the obtained lactide to ring-opening polymerization, through which lactide can be efficiently obtained. The present invention relates to a lactide production apparatus for continuously or intermittently producing lactide, comprising: a depolymerization device 11 for subjecting lactic acid oligomers to a depolymerization reaction; a catalyst regeneration device 27 for subjecting a residue in the depolymerization device to a depolymerization reaction in order to reduce the molecular weights of lactic acid oligomers contained in the residue; and a distillation column 29 for condensing lactide generated in a gas form as a result of the depolymerization reaction.

## Description

### Technical Field

The present invention relates to a method and an apparatus for producing lactide and also relates to a method and an apparatus for producing polylactic acid with the use thereof.

### Background Art

Polylactic acid is an aliphatic polyester produced with the use of lactic acid as a starting material. Examples of known methods for synthesizing polylactic acid include methods wherein lactic acid is concentrated such that the moisture contained therein is reduced, followed by condensation. Accordingly, lactic acid oligomers are generated. Such oligomers are temporally depolymerized with the addition of a catalyst such as antimony oxide for generation of cyclic dimers (lactides). If necessary, purification is carried out via crystallization, or the like. Thereafter, ring-opening polymerization is caused with the addition of a catalyst such as tin octylate to lactides.

In some cases, the content of moisture regarded as an impurity in lactic acid monomer is approximately 10% to 15%. Therefore, in the concentration step, in order to facilitate the initiation of esterification treatment between monomers, moisture removal is carried out. In this concentration step, such moisture is removed by heating at 120°C to 250°C and, if necessary, depressurization using a vacuum pump or the like.

In the condensation step, water generated in reaction of esterification between lactic acid monomers is removed by vaporization caused by heating at 120°C to 250°C under vacuum condition created with the use of a vacuum pump or the like and desirably under vacuum condition at 10 torr or less. Unlike depressurization in the concentration step, depressurization in the above step is an essential condition for promoting a reaction of esterification. As a result of the condensation step, lactic acid oligomers are generated from lactic acid monomers.

Lactic acid oligomers generated in the condensation step are subjected to a depolymerization step in which oligomers come into contact with a depolymerization catalyst such as tin octylate or antimony trioxide under heating at 120°C to 250°C under vacuum condition created with the use of a vacuum pump or the like and desirably under vacuum condition at 100 torr or less. This results in generation of cyclic dimers of lactic acid (lactides). In general, the thus generated lactides is usually in the form of a gas in the environment in the depolymerization step and can be recovered via cooling and condensation.

A variety of methods for producing lactide for polylactic acid synthesis have been developed. For example, a lactide production apparatus having a lactide reactor for depolymerizing prepolymers including lactic acid oligomers, such apparatus capable of reusing a portion of non-reactive high-boiling polylactic acid contained in a lactide reactor or a purge flow of a different nonvolatile impurity in a device located upstream of the lactide reactor or supplying the same for polymerization to a device located downstream of the lactide reactor, has been reported (Patent Document 1). This apparatus can reflux a reaction solution (containing lactic acid oligomers and a catalyst; hereinafter referred to as "residue") discharged from a lactide reactor to the device used in the depolymerization step, to the concentration step or condensation step before the depolymerization step, or to the same to the device used in the ring-opening polymerization step after the depolymerization step.

In the case of the apparatus disclosed in Patent Document 1, the yield is improved by feeding the residue back to the lactide reactor inlet. However, a ring-opening polymerization reaction simultaneously takes place as a reverse reaction, resulting in an increase in the molecular weight. Accordingly, the residue viscosity is higher than the initial viscosity of lactic acid oligomers. Therefore, the activity of a catalyst contained in the residue serves as a diffusion rate controlling factor, resulting in an apparent decrease in the reaction rate. The depolymerization reaction rate decreases. As a result, the operation of a plant becomes unstable, which is problematic. When the residue is refluxed to the concentration step or the condensation step, a catalyst contained in the residue inhibits a condensation reaction, resulting in a decrease in the molecular weights of lactic acid oligomers. This is problematic. When the residue is fed to the ring-opening polymerization step, low-molecular-weight lactic acid oligomers are mixed in polylactic acid, resulting in a wider molecular weight distribution. Accordingly, for example, the degree of crystallization decreases, resulting in deterioration of polymer physical properties. This is problematic.
Patent Document 1: JP Patent No. 3258324

### Disclosure of the Invention

The present invention has been made in view of the above problems. It is an object of the present invention to provide a method and an apparatus for producing polylactic acid by depolymerizing lactic acid oligomers and subjecting the obtained lactide to ring-opening polymerization, through which lactide can be efficiently obtained.

As a result of intensive studies in order to achieve the above object, the present inventors have found that a depolymerization reaction can proceed with good efficiency by subjecting a highly viscous residue remaining in a depolymerization device to a depolymerization reaction again, thereby reducing the molecular weights of lactic acid oligomers contained in the residue so as to decrease the viscosity of the residue. This has led to the completion of the present invention.

Specifically, the present invention encompasses the following inventions.
(1) A method for continuously or intermittently producing lactide by depolymerizing lactic acid oligomers, comprising the steps of:
   a) subjecting lactic acid oligomers to a depolymerization reaction for synthesizing lactide in a depolymerization device;
   b) subjecting a residue in the depolymerization device to a depolymerization reaction in a reaction vessel having an evaporation area per unit volume of solution larger than that of the reaction vessel of the depolymerization device, thereby reducing the molecular weights of lactic acid oligomers contained in the residue; and
   c) recovering lactide generated in a gas form as a result of the depolymerization reaction via condensation.
(2) The method according to (1), further comprising a step of feeding the lactic acid oligomers with reduced molecular weights that have been generated in step "b" back to the depolymerization device.
(3) The method according to (1) or (2), wherein the residue in a molten form is formed into a thin layer with external force in step "b."
(4) The method according to any one of (1) to (3), wherein a depolymerization reaction is carried out with the use of a centrifugal thin film evaporator in step "b."
(5) The method according to any one of (1) to (4), further comprising a step of synthesizing lactic acid oligomers via condensation of lactic acid.
(6) A method for producing polylactic acid, comprising producing lactide by depolymerizing lactic acid oligomers by the method according to any one of (1) to (5) and subjecting the obtained lactide to ring-opening polymerization.
(7) A lactide production apparatus for continuously or intermittently producing lactide, comprising:
   a depolymerization device for subjecting lactic acid oligomers to a depolymerization reaction;
   a catalyst regeneration device, which has a reaction vessel having an evaporation area per unit volume of solution larger than that of the reaction vessel of the depolymerization device and is for subjecting a residue in the depolymerization device to a depolymerization reaction in order to reduce the molecular weights of lactic acid oligomers contained in the residue; and
   a distillation column for condensing lactide generated in a gas form as a result of the depolymerization reaction.
(8) The apparatus according to (7), further comprising a liquid-feeding pump for feeding lactic acid oligomers with decreased molecular weights generated in the catalyst regeneration device to the depolymerization device.
(9) The apparatus according to (7) or (8), wherein the catalyst regeneration device has a means for forming a thin layer of the residue in a molten form.
(10) The apparatus according to any one of (7) to (9), wherein the catalyst regeneration device is a centrifugal thin film evaporator.
(11) The apparatus according to any one of (7) to (10), further comprising a lactic acid condensation device for producing lactic acid oligomers via condensation of lactic acid.
(12) A polylactic acid production apparatus for continuously or intermittently producing polylactic acid, comprising:
   the lactide production apparatus according to any one of (7) to (11);
      and
   a ring-opening polymerization device for ring-opening polymerization of lactide.

According to the present invention, the efficiency of the use of a catalyst is improved, and therefore the depolymerization reaction rate increases. Accordingly, lactide can be efficiently produced and the quality of polylactic acid can also be improved.

This description includes part or all of the contents as disclosed in the description and drawings of Japanese Patent Application No. 2007-289664, which is a priority document of the present application.

### Brief Description of the Drawings

Fig. 1 schematically shows an embodiment of the polylactic acid production apparatus of the present invention.
Fig. 2 schematically shows a centrifugal thin film evaporator.
Fig. 3 schematically shows an embodiment of the polylactic acid production apparatus of the present invention.

Explanation of Reference Numerals:
1: Lactic acid supply device; 2: Liquid-feeding pump; 3: Lactic acid concentration device; 4: Liquid-feeding pump; 5: Concentrated lactic acid buffer tank; 6: Liquid-feeding pump; 7: Lactic acid condensation device; 8: Liquid-feeding pump; 9: Oligomer buffer tank; 10: Liquid-feeding pump; 11: Depolymerization device; 12: Distillation column; 13: Upper distillation column; 14: Liquid-feeding pump; 15: Lactide purification device; 16: Liquid-feeding pump; 17: Ring-opening polymerization device; 18: Distillation column; 19: Condenser; 20: Vacuum pump; 21: Distillation column; 22: Condenser; 23: Vacuum pump; 24: Condenser; 25: Subsequent condenser; 26: Vacuum pump; 27: Catalyst regeneration device; 28: Distillation column; 29: Upper distillation column; 30: Condenser; 31: Subsequent condenser; 32: Vacuum pump; 33: Liquid-feeding pump; 34: Catalyst regeneration device; 35: Distillation column; 36: Upper distillation column; 37: Condenser; 38: Subsequent condenser; and 39: Vacuum pump.

### Best Mode for Carrying Out the Invention

According to the present invention, the concept of "lactic acid oligomers" refers to from lactic acid dimers to lactic acid polymer having molecular weights of approximately 50,000. The molecular weight of a lactic acid oligomer that is introduced into a depolymerization device for lactide production is generally 150 to 10,000, preferably 500 to 5,000 in a number-average molecular weight.

According to the present invention, the term "lactide" refers to a cyclic ester generated by dehydrating 2 water molecules from 2 lactic acid molecules. Lactic acid includes both L-lactic acid and D-lactic acid.

The term "polylactic acid" refers to a polymer containing lactic acid as a main component. Examples of polylactic acid include poly L-lactic acid homopolymers, poly D-lactic acid homopolymers, poly L/D-lactic acid copolymer substances, polylactic acid copolymers obtained by copolymerizing the above polylactic acids with other ester bond-forming components such as hydroxycarboxylic acid, lactones, dicarboxylic acid, and diol, and mixtures of such homopolymers or copolymers and additives serving as minor constituents. Examples of additives include antioxidants, stabilizers, ultraviolet absorbing agents, pigments, colorants, inorganic particles, a variety of fillers, release agents, plasticizers, and other similar substances. The additive rates of these copolymer components and additive agents can be arbitrarily determined. However, a major component thereof is lactic acid or a lactic acid-derived substance. In addition, a copolymer component and an additive agent preferably do not account for more than 50% by weight, and particularly preferably more than 30% by weight, of the total.

According to the present invention, the expression "continuously or intermittently" is used with the meaning generally used in the art. It indicates a case in which the time of supplying starting materials at least partially overlaps the time of discharging products or a case in which starting materials are continuously or intermittently supplied and products are continuously or intermittently discharged.

The method for producing lactide of the present invention comprises the steps of:
a) subjecting lactic acid oligomers to a depolymerization reaction for synthesizing lactide in a depolymerization device;
b) subjecting a residue in the depolymerization device to a depolymerization reaction in a reaction vessel having an evaporation area per unit volume of solution larger than that of the reaction vessel of the depolymerization device, thereby reducing the molecular weight of lactic acid oligomer contained in the residue; and
c) recovering lactide generated in a gas form as a result of the depolymerization reaction via condensation.

The method for producing lactide can be carried out with the use of a lactide production apparatus comprising:
a depolymerization device for subjecting lactic acid oligomers to a depolymerization reaction;
a catalyst regeneration device, which has a reaction vessel having an evaporation area per unit volume of solution larger than that of the reaction vessel of the depolymerization device and is for subjecting a residue in the depolymerization device to a depolymerization reaction in order to reduce the molecular weights of lactic acid oligomers contained in the residue; and
a distillation column for condensing lactide generated in a gas form as a result of the depolymerization reaction.

The depolymerization device has at least a reactor, a lactic acid oligomer feeding opening, a lactide discharge opening, and a residue discharge opening. In addition, a thermometer is usually provided thereto. The reactor that can be used is not particularly limited and can be a vertical reactor, a horizontal reactor, or a tank reactor. Examples of an agitating blade that can be used include paddle blades, turbine blades, anchor blades, double-motion blades, and helical ribbon blades.

As a method for heating a reactor, methods that are generally used in the art can be used. Examples of such methods include a method wherein a heat medium jacket is provided to the outer peripheral part of a reactor such that a reaction solution is heated by heat transfer through the reactor wall and a method wherein heating is carried out via heat transfer through a heat medium provided inside a rotary shaft of the agitating blade. These methods may be used alone or in combinations.

In general, the depolymerization device comprises a vacuum pump. A depolymerization reaction of lactic acid oligomers is carried out under vacuum condition generally at 100 torr or less and preferably at 10 torr or less by heating generally at 120°C to 250°C and preferably at 120°C to 200°C. As a result of the depolymerization reaction, lactide is generated in the form of a gas.

For a depolymerization reaction, if necessary, a catalyst for a depolymerization reaction may be added. Conventionally known catalysts can be used. Examples thereof include catalysts comprising: metals selected from the group consisting of metals of Groups IA, IIIA, IVA, IIB, and VA of the periodic table; and metal compounds thereof.

Examples of catalysts belonging to Group IA include hydroxides of alkali metals (e.g., sodium hydroxide, potassium hydroxide, and lithium hydroxide), salts of alkali metals and weak acids (e.g., sodium lactate, sodium acetate, sodium carbonate, sodium octylate, sodium stearate, potassium lactate, potassium acetate, potassium carbonate, and potassium octylate), alkoxides of alkali metals (e.g., sodium methoxide, potassium methoxide, sodium ethoxide, and potassium ethoxide).

Examples of catalysts belonging to Group IIIA include aluminium ethoxide, aluminium isopropoxide, alumina, and aluminium chloride.

Examples of catalysts belonging to Group IVA include organotin-based catalysts (e.g., tin 2-ethylhexanoate, tin lactate, tin tartrate, tin dicaprylate, tin distearate, tin dioleate, tin α-naphthoate, tin β-naphthoate, and tin octylate), powdered tin, oxidized tin, and halogenated tin.

Examples of catalysts belonging to Group IIB include zinc powder, halogenated zinc, oxidized zinc, and organozinc-based compounds.

Examples of catalysts belonging to Group IVB include titanium-based compounds such as tetrapropyl titanate and zirconium-based compounds such as zirconiumisopropoxide.

Among the above, it is preferable to use a tin-based compound such as tin octylate or an antimony-based compound such as antimony trioxide.

In addition, the content of catalyst used is approximately 0.01% to 20% by weight, preferably approximately 0.05% to 15% by weight, and more preferably approximately 0.1% to 10% by weight based on the weight of lactic acid oligomers.

In the depolymerization device, a lactic acid oligomer depolymerization reaction and a ring-opening polymerization reaction that is a reverse reaction of such depolymerization simultaneously proceeds. Therefore, as the reactions proceed, molecular weights of lactic acid oligomers increase and the viscosity of the reaction solution increases. When the viscosity of the reaction solution increases, the dispersibility of a depolymerization catalyst mixed in the reaction solution deteriorates, resulting in a decrease in the rate of depolymerization reaction. Accordingly, the lactide production efficiency decreases. Therefore, the residue containing lactic acid oligomers with increased molecular weights is discharged and subjected to a depolymerization reaction in a different reaction device such that the molecular weights of lactic acid oligomers can be reduced. A depolymerization reaction for reducing the molecular weights of lactic acid oligomers is carried out under pressure and temperature conditions similar to those used in a depolymerization device located upstream of the depolymerization device. The depolymerization reaction is carried out in a reaction vessel having an evaporation area in terms of the unit solution amount larger than that of a reaction vessel of a depolymerization device located upstream of the depolymerization device.

Lactide produced from the residue can be rapidly degassed by subjecting the residue to a depolymerization reaction in a reaction device with a reaction vessel having an evaporation area per unit volume of solution (specific surface area) amount larger than that of the reaction vessel of the depolymerization device. As a result, due to chemical equilibrium, the rate of ring-opening polymerization reaction decreases and the molecular weight increasing effects are reduced. Then, the molecular weights of lactic acid oligomers contained in the residue decrease. When the molecular weights of lactic acid oligomers contained in the residue decrease, the viscosity decreases. Accordingly, the catalyst activity becomes less likely to be influenced by the diffusion rate, resulting in an increase in the catalyst activity. Therefore, the rate of depolymerization reaction is improved. In view of the above, the term "catalyst regeneration device" used herein refers to a reaction device used for a step of decreasing the molecular weights of lactic acid oligomers contained in the residue; that is to say, a reaction device having a reaction vessel with an evaporation area per unit volume of solution amount (specific surface area) larger than that of the reaction vessel of the depolymerization device.

Herein, the term "residue" refers to a reaction solution which has a high viscosity and contains lactic acid oligomers with relatively high molecular weights and a depolymerization catalyst, and is remaining in a reactor as a result of a depolymerization reaction in a depolymerization device. More specifically, it refers to a reaction solution containing lactic acid oligomers with number-average molecular weights of generally 5000 to 30000 and preferably 5000 to 10000. The residue may contain unreacted lactic acid, polylactic acid, water, lactide, and the like, in addition to lactic acid oligomers and a catalyst. The residue may be removed from a depolymerization device and feed it to a catalyst regeneration device in an amount such that "the mass of the lactide oligomers introduced into a depolymerization device - the mass of generated lactide = the residue mass".

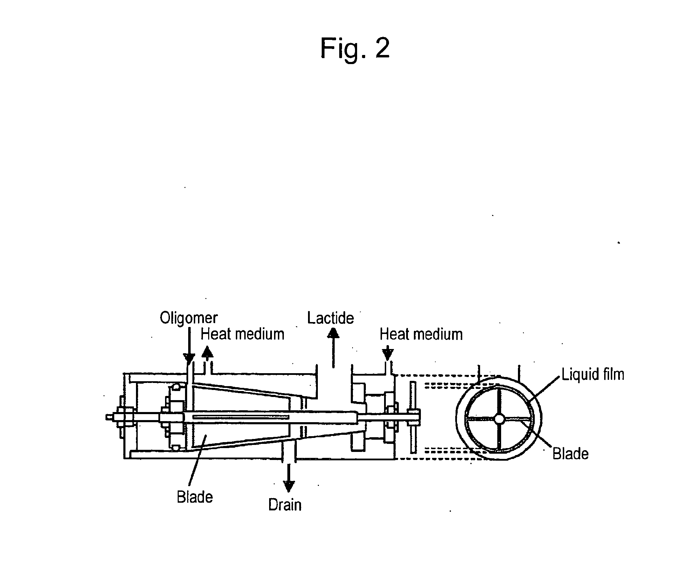

Any catalyst regeneration device can be used as long as it has a specific surface area larger than that of the depolymerization device. However, it has at least a reactor, a residue feeding opening, a lactide discharge opening, and a residue discharge opening. In addition, a thermometer is generally provided to the device. The reactor can be either a vertical reactor or a horizontal reactor. Preferably, the device has a means of forming the residue (in a molten form) into a thin layer with external force. A means for forming the residue (in a molten form) into a thin layer with external force is, in other words, a means for applying centrifugal force. Specifically, a rotor having blades is used as such means. An example of a catalyst regeneration device is a centrifugal thin film evaporator or the like. According to the present invention, the term "centrifugal thin film evaporator" refers to a device for applying centrifugal force generated by rotating blades in a fixed apparatus casing. The reaction solution supplied to the device comes into contact with the internal wall of the device casing via centrifugal force for liquid film formation. Then, evaporation is induced by heating the device casing from the external side. Centrifugal force is applied to lactic acid oligomers via rotating blades in the fixed device casing to which a rotor with blades has been installed. A centrifugal thin film evaporator has advantageous features that, for example, the facility scale can be reduced and the liquid film thickness can be controlled by controlling the gap width between the blade and the casing and the rotation of the blades. Fig. 2 schematically shows a centrifugal thin film evaporator. In general, plate blades are installed in parallel to a rotary shaft in a manner such that they radially extend from the rotary shaft. In some cases, the blade that is twisted to results in a screw form is used. A centrifugal thin film evaporator having a horizontal or vertical rotary shaft or a rotary shaft at an angle between horizontal and vertical may be used.

In a horizontal centrifugal thin film evaporation device, a catalyst can be retained in an apparatus such that the catalyst can be dispersed in molten lactic acid oligomers by operating the rotor and the blades. Therefore, even in a case in which molten lactic acid oligomers are continuously supplied from one end of the device, it is possible to maintain a certain retention amount if the supply rate corresponds to the amount of lactide vapor discharged. Therefore, continuous discharge from the other end is not necessarily required. In a case in which the retention amount increases due to accumulation of the residue generated after depolymerization of molten lactic acid oligomers, the solution in the device is intermittently discharged and it is possible to resume the operation by adding a new catalyst to the device.

In particular, the centrifugal thin film evaporator has the following advantageous features. First, since it has a high evaporation capacity, it is appropriate for processing of a relatively highly viscous substance. This is because the solution is formed into a uniform thin film by rotor blades and is intensively agitated. Even in a case of highly viscous substance, it a high heat transfer coefficient can be realized. Next, since it is possible to shorten the retention time, it is appropriate for processing of a thermally unstable substance. This is because the solution in the form of a thin film is processed such that a desired concentration is realized within a very short period of time. In addition, it can be used for vacuum evaporation and is appropriate for processing of a thermally unstable substance or a high boiling point substance. This is because, since the solution in the form of a thin film is processed, the boiling point does not increased at a liquid head and thus evaporation can be performed at a boiling point at the degree of vacuum in the system. In addition, adhesion of scales (adhering matter) does not take place, allowing continuous operation. This is because the solution is agitated by rotor blades such that a portion of the solution forming the heating surface is always replaced by a new portion. Further, droplets dispersed in the centrifugal thin film evaporator are hit by the rotor blades such that the droplets are pressed to the liquid film formed on the rotor blades by centrifugal force so as to be absorbed by the film. Accordingly, it is possible to prevent dispersion of droplets of a catalyst in the catalyst regeneration device and discharge of the same from the device.

According to the present invention using a centrifugal thin film evaporator as a catalyst regeneration device, an apparatus can be down-sized since it has an evaporation area larger than a tank-type reactor relative to the apparatus volume. In addition, the temperature of lactic acid oligomers can be readily increased during a short period of time and therefore it is possible to complete a depolymerization reaction within several to several tens of minutes. Since a liquid film is thin, vaporization of generated lactide is extremely quick. Therefore, there are few optical isomers generated by an isomerization reaction of lactic acid oligomers and impurities generated as a result of thermal decomposition of lactic acid oligomers. Accordingly, lactide can be obtained at a high purity. In addition, the rate of depolymerization reaction becomes faster than that of ring-opening polymerization reaction, resulting in a decrease in the molecular weights of lactic acid oligomers and then a decrease in the viscosity. As a result, catalyst activity is restored and a depolymerization reaction is further promoted.

In some cases, the residue discharged from the catalyst regeneration device is fed back to the depolymerization device with the use of liquid feeding pump or the like, and the reaction yield can be further improved. The residue discharged from the catalyst regeneration device is a reaction solution containing lactic acid oligomers with lowered molecular weights and a catalyst for a depolymerization reaction. It can also contain lactic acid, water, polylactic acid, lactide, and the like. The residue has a low viscosity, indicating recovery of catalyst activity. Lactic acid oligomers with lowered molecular weights contained in the residue have number-average molecular weights of generally 2000 to 10000 and preferably 2000 to 5000.

In general, the catalyst regeneration device is provided with a vacuum pump. A depolymerizing reaction the residue is carried out under vacuum condition at generally 100 torr or less and preferably 10 torr or less by heating at generally 120°C to 250°C and preferably 120°C to 200°C. As a result of the depolymerization reaction, the molecular weights of oligomers decrease and the residue viscosity decreases. Accordingly, the activity of the catalyst contained in the residue is restored, resulting in promotion of the depolymerization reaction. Lactide is generated in the form of a gas. Further, it is advantageous when a continuous reaction is carried out since a reaction solution in the depolymerization device does not become excessively viscous.

When a depolymerization reaction proceeds in a catalyst regeneration device and lactide is evaporated, the catalyst concentration relatively increases, resulting in a decrease in the optical purity of lactide. This is because a decrease in the optical purity of lactide is proportional to the catalyst concentration and the retention time. Therefore, if necessary, it is possible to discharge and discard the residue when the residue viscosity has decreased to a certain level or to reflux the residue to the depolymerization device. A plurality of catalyst regeneration devices may be installed to the system. In such case, the residue discharged from a catalyst regeneration device located at most downstream can be refluxed to the depolymerization device or a catalyst regeneration device located immediately upstream of the catalyst regeneration device located most downstream. Preferably, the specific surface area of the catalyst regeneration device increases toward the device located most downstream.

Vapor containing lactide generated in the form of a gas in the depolymerization device or catalyst regeneration device is discharged outside the depolymerization device and introduced into a distillation column. In the distillation column, lactide is condensed so as to be recovered and then transferred to preferably a lactide purification device. It is possible to condense lactic acid oligomers alone in the first distillation column so as to allow lactide vapor to pass therethrough and to recover lactide in the subsequent distillation or the like (in the upper distillation column) by adequately controlling the refrigerant temperature. The lactic acid oligomers condensed above may be refluxed so as to be fed back to the lactic acid condensation device, the lactic acid concentration device, the depolymerization device, the catalyst regeneration device, or the like.

A desired example of a distillation column is a surface condenser in which vapor and a refrigerant indirectly come into contact with each other via metal tubes. This is because acid generation takes place due to lactide decomposition caused by direct contact between lactide and a water-containing refrigerant. As a result of acid generation, acid serves as a catalyst to inhibit the progress in a ring-opening polymerization reaction. In addition, acid may cause corrosion of materials for a distillation column and the like. There are exceptional cases in which a refrigerant that is inert in the presence of lactide and lactic acid oligomers is used. In such case, it is necessary to sufficiently dry a refrigerant to reduce the hygroscopic moisture therein.

Lactic acid oligomers separated in the distillation column may be fed back to any one of the lactic acid concentration device, the concentrated lactic acid buffer tank, the lactic acid condensation device, the oligomer buffer tank, the depolymerization device inlet and the like. However, lactic acid oligomers separated from lactide in the distillation column have very small molecular weights as a result of a depolymerization reaction in some cases. It is not desirable to introduce such low-molecular-weight lactic acid oligomers into the depolymerization device directly. It is desirable to condense the oligomers again so as to improve the molecular weights and to improve the lactide yield. Specifically, it is desirable to feed the low-molecular-weight lactic acid oligomers back to the lactic acid condensation device so as to subject the oligomers to a condensation reaction. It is also possible to use a plurality of distillation columns in order to reflux lactic acid oligomers that have been separated according to their molecular weights. Then, it is desirable to feed the low-molecular-weight lactic acid oligomers back to at least one of the lactic acid concentration device, the lactic acid oligomer buffer tank, and the lactic acid condensation device and to feed high-molecular-weight lactic acid oligomers back to at least one of the lactic acid condensation device, the lactic acid oligomer buffer tank, and the depolymerization device inlet.

The method for producing lactide of the present invention may further comprise a step of synthesizing a lactic acid oligomer used as a starting material; that is to say, a step of synthesizing a lactic acid oligomer by condensing lactic acid. In such case, any type of lactic acid may be used as long as it is produced by a conventional method. However, it is preferable to use lactic acid with a low moisture content. With the use of such lactic acid, it becomes possible to omit a step for evaporating contained moisture in order to concentrate lactic acid, which is advantageous in terms of costs.

Preferably, the moisture originally contained in lactic acid can be removed by evaporation with heating. The moisture contained in lactic acid used as a starting material may be removed together with the moisture generated through a lactic acid condensation reaction in the lactic acid condensation step. Alternatively, it is also possible to preliminarily remove the moisture from lactic acid used as a starting material for concentration of lactic acid and then subject the resultant to the lactic acid condensation step.

In the former case, lactic acid used as a starting material is directly transport to a lactic acid condensation device for a lactic acid condensation reaction. Meanwhile, in the latter case, a lactic acid concentration device and a lactic acid condensation device are connected in series. Lactic acid is heated in the lactic acid concentration device located upstream of the lactic acid condensation device for evaporation of the moisture and then the obtained lactic acid concentration product is transported to the lactic acid condensation device for a lactic acid condensation reaction. It is also possible to install a lactic acid supply device upstream of the lactic acid condensation device or the lactic acid concentration device and to supply lactic acid from the lactic acid supply device to any one of them.

Lactic acid oligomers are generated by heating lactic acid in the lactic acid condensation device. In a lactic acid condensation reaction, the molecular weights of lactic acid oligomers to be obtained are adjusted to the molecular weights at which the oligomers can be adequately introduced into the above depolymerization device. Specifically, a reaction is carried out in a manner such that the number-average molecular weight of lactic acid oligomers obtained is generally 150 to 10,000 and preferably 500 to 5,000. The lactic acid condensation reaction is generally carried out at a pressure of 100 torr or less, preferably 10 torr or less, and more preferably 1 torr or less, and generally at a temperature of 160°C to 220°C and preferably 170°C to 200°C with a gradual temperature increase. Thermal decomposition of lactic acid and lactic acid oligomers can be inhibited by minimize the heating time.

During the lactic acid condensation reaction, if necessary, a catalyst for a lactic acid condensation reaction may be added. A conventionally known catalyst can be used as a catalyst for such reaction. Examples thereof include: organotin-based catalysts (e.g., tin lactate, tin tartrate, tin dicaprylate, tin dilaurate, tin dipalmitate, tin distearate, tin dioleate, tin α-naphthoate, tin β-naphthoate, and tin octylate); and powdered tin. In a case in which the above lactic acid supply device is installed, it is possible to preliminarily add such catalyst to the lactic acid supply device.

The lactic acid condensation device has at least a reactor, a feeding opening, and a discharge opening. In addition, a vacuum pump for depressurizing inside the reactor and a thermometer are usually provided thereto. The reactor is not particularly limited and can be a vertical reactor, a horizontal reactor, or a tank reactor. Examples of an agitating blade that can be used include paddle blades, turbine blades, anchor blades, double-motion blades, and helical ribbon blades.

As a method for heating a reactor, methods that are generally used in the art can be used. Examples of such methods include a method wherein a heat medium jacket is provided to the outer peripheral part of a reactor such that a reaction solution is heated by heat transfer through the reactor wall and a method wherein heating is carried out via heat transfer through a heat medium provided inside a rotary shaft of the agitating blade. These methods may be used alone or in combinations. These methods may be used alone or in combination.

Lactic acid oligomers obtained through a condensation reaction may be temporarily accumulated in the lactic acid oligomer supply device used as a buffer tank and then transported to a depolymerization device or directly transported to a depolymerization device. When a depolymerization reaction is continuously carried out, it is preferable to accumulate lactic acid oligomers in the lactic acid oligomer supply device and then continuously transport the oligomers to the depolymerization device.

One embodiment of the present invention relates to a method for producing polylactic acid, comprising producing lactide by the above method and subjecting the obtained lactide to ring-opening polymerization.

In the ring-opening polymerization device, a ring-opening polymerization reaction of lactide is carried out by heating in an inert gas atmosphere at generally 120°C to 250°C and preferably 120°C to 200°C. As a result of the ring-opening polymerization reaction, polylactic acid is generated. The molecular weight of polylactic acid obtained through a ring-opening polymerization reaction is generally 100,000 to 500,000 and preferably 200,000 to 300,000 in terms of the weight-average molecular weight.

The ring-opening polymerization device has at least a reactor, a lactide feeding opening, and a polylactic acid discharge opening. In addition, a thermometer is usually provided thereto. The reactor is not particularly limited and can be a vertical reactor, a horizontal reactor, or a tank reactor. Two or more reactors may be installed in series. Examples of an agitating blade that can be used include paddle blades, turbine blades, anchor blades, double-motion blades, and helical ribbon blades.

As a method for heating a reactor, methods that are generally used in the art can be used. Examples of such methods include a method wherein a heat medium jacket is provided to the outer peripheral part of a reactor such that a reaction solution is heated by heat transfer through the reactor wall and a method wherein heating is carried out via heat transfer through heat transfer tubes (coils) provided inside the reactor. These methods may be used alone or in combinations.

For a ring-opening polymerization reaction, if necessary, a catalyst for a depolymerization reaction may be used. Conventionally known catalysts can be used. Examples such catalysts that can be used include catalysts comprising: metals selected from the group consisting of metals of Groups IA, IIIA, IVA, IIB, and VA of the periodic table; and metal compounds thereof.

Examples of catalysts belonging to Group IA include hydroxides of alkali metals (e.g., sodium hydroxide, potassium hydroxide, and lithium hydroxide), salts of alkali metals and weak acids (e.g., sodium lactate, sodium acetate, sodium carbonate, sodium octylate, sodium stearate, potassium lactate, potassium acetate, potassium carbonate, and potassium octylate), alkoxides of alkali metals (e.g., sodium methoxide, potassium methoxide, sodium ethoxide, and potassium ethoxide).

Examples of catalysts belonging to Group IIIA include aluminium ethoxide, aluminium isopropoxide, alumina, and aluminium chloride.

Examples of catalysts belonging to Group IVA include organotin-based catalysts (e.g., tin lactate, tin tartrate, tin dicaprylate, tin distearate, tin dioleate, tin α-naphthoate, tin β-naphthoate, and tin octylate), powdered tin, oxidized tin, and halogenated tin.

Examples of catalysts belonging to Group IIB include zinc powder, halogenated zinc, oxidized zinc, and organozinc-based compounds.

Examples of catalysts belonging to Group IVB include titanium-based compounds such as tetrapropyl titanate and zirconium-based compounds such as zirconiumisopropoxide.

Among the above, it is preferable to use a tin-based compound such as tin octylate or an antimony-based compound such as antimony trioxide.

In addition, the content of catalyst used is approximately 1 to 2000 ppm, preferably approximately 5 to 1500 ppm, and more preferably approximately 10 to 1000 ppm based on the weight of lactide.

For a ring-opening polymerization reaction, if necessary, a polymerization initiator used for ring-opening polymerization reaction may be added for the purpose of, for example, adjusting the molecular weight. Examples of polymerization initiators that can be used include alcohols such as 1-dodecanol, which is a substance having a hydroxyl group.

The lactide production apparatus and the polylactic acid production apparatus of the present invention can be structured in a manner generally used in the art. Those skilled in the art can adequately combine other necessary apparatuses with the apparatus of the present invention. Therefore, the lactide production apparatus of the present invention may comprise a lactic acid supply device, a lactic acid concentration device, a concentrated lactic acid buffer tank, an oligomer buffer tank, a upper distillation column, and a lactide purification device, in addition to a depolymerization device, a catalyst regeneration device, a distillation column, a liquid-feeding pump, and a lactic acid condensation device described above.

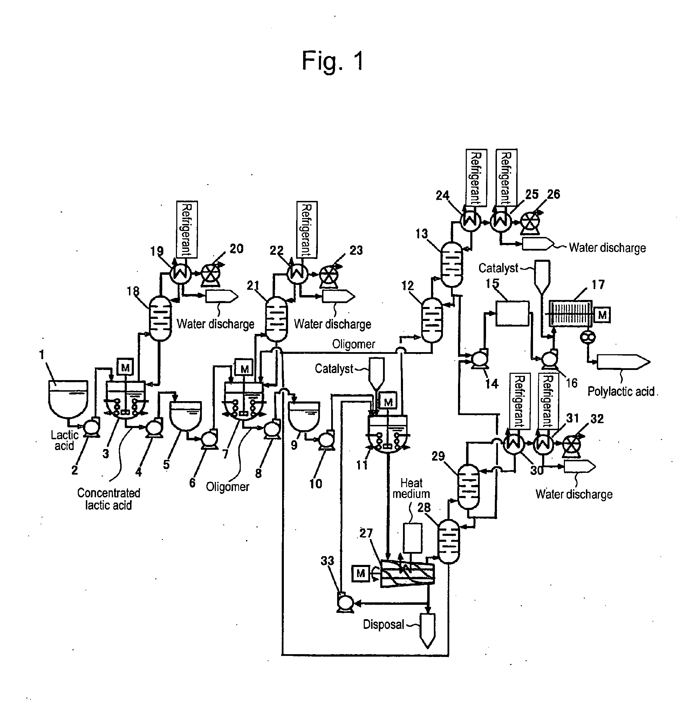

Fig. 1 shows one embodiment of a polylactic acid production apparatus, including the lactide production apparatus of the present invention. The apparatus is composed of a lactic acid supply device 1, a liquid-feeding pump 2, a lactic acid concentration device 3, a liquid-feeding pump 4, a concentrated lactic acid buffer tank 5, a liquid-feeding pump 6, a lactic acid condensation device 7, a liquid-feeding pump 8, an oligomer buffer tank 9, a liquid-feeding pump 10, a depolymerization device 11, a distillation column 12, a upper distillation column 13, a liquid-feeding pump 14, a lactide purification device 15, a liquid-feeding pump 16, and a ring-opening polymerization device 17, which are provided in such order starting from the upstream side of the reaction path. The apparatus is further composed of a catalyst regeneration device 27, a distillation column 28, and a upper distillation column 29, which are provided in such order after a residue discharge opening of a depolymerization device 11.

The polylactic acid production apparatus shown in fig. 1 is used for producing polylactic acid by evaporating the moisture contained in lactic acid in the lactic acid concentration device so as to obtain concentrated lactic acid, condensing concentrated lactic acid in the lactic acid condensation device so as to generate lactic acid oligomers, and depolymerizing the obtained lactic acid oligomers in the depolymerization device under reduced pressure for generation of lactide, and then subjecting purified lactide to a ring-opening polymerization.

Lactic acid is supplied from the lactic acid supply device 1 and the moisture is removed in the lactic acid concentration device 3 to obtain concentrated lactic acid. The concentrated lactic acid is supplied to the lactic acid condensation device 7 via the concentrated lactic acid buffer tank 5 and then formed into lactic acid oligomers through a condensation reaction. Lactic acid oligomer is sent to the depolymerization device 11 via the oligomer buffer tank 9 such that lactide is obtained through a depolymerization reaction. Lactide is separated from the oligomers in the distillation column 12 and then condensed and recovered in the upper distillation column 13. Lactide discharged from the lactide purification device 15 is transferred to the ring-opening polymerization device 17. In the ring-opening polymerization device 17, a reaction of ring-opening polymerization of lactide is carried out in an inert gas atmosphere. Thus, polylactic acid is produced. The residue discharged from the depolymerization device 11 is transferred to the catalyst regeneration device 27. Lactide discharged from the catalyst regeneration device 27 is transferred to the ring-opening polymerization device 17 via the distillation column 28 and the upper distillation column 29.

Hereinafter, the embodiment shown in fig. 1 is specifically described.

In the lactic acid concentration device 3, the moisture contained in lactic acid is evaporated by heating. Heating is carried out at 120°C to 150°C in the presence of a flow of nitrogen gas. In a lactic acid concentration reaction, moisture and lactic acid are formed into a gas. The thus formed gas enters the distillation column 18. Then lactic acid is removed from the gas and then refluxed into the lactic acid concentration device 3.

Concentrated lactic acid produced in the lactic acid concentration device 3 is sent to the lactic acid condensation device 7 via the concentrated lactic acid buffer tank 5.

In the lactic acid condensation device 7, a lactic acid condensation is allowed to proceed. Then, the moisture formed as a result of the reaction is evaporated. The reaction is carried out under vacuum pressure of 10 torr or less and at a temperature of 120°C to 250°C. In the lactic acid condensation reaction, a gas containing the moisture, lactic acid, low-molecular-weight lactic acid oligomers, and lactide formed as a result of decomposition of lactic acid oligomers is formed The gas is transferred from the lactic acid condensation device 7 to the vacuum pump 23 and enters the distillation column 21. Then, lactic acid, low-molecular-weight lactic acid oligomer, and lactide are removed from the gas and refluxed into the lactic acid condensation device 7.

Lactic acid oligomers generated in the lactic acid condensation device 7 are sent to the depolymerization device 11.

In the depolymerization device 11, an oligomer depolymerization reaction is allowed to proceed. The reaction is carried out by allowing lactic acid oligomers to come into contact with a depolymerization catalyst under vacuum pressure of 10 torr or less and at a temperature of 120°C to 250°C. Lactide in the form of a gas generated as a result of the reaction is sent to the distillation column 12.

In the distillation column 12, lactide in the form of a gas is cooled such that impurities contained in lactide, such as lactic acid oligomers, are liquefied and lactide in the form of a gas is sent to the upper distillation column 13. Most of the lactic acid oligomers that have been separated from lactide have smaller molecular weights as a result of a depolymerization reaction. In order to improve the yield of lactide, it is desirable to subject such low-molecular-weight lactic acid oligomers again to condensation for use. Then, the resultant is refluxed into the lactic acid condensation device 7.

Lactide in the form of a gas is cooled and condensed in the upper distillation column 13 and then sent to the lactide purification device 15. The gas separated from lactide having a high water vapor content enters the condenser 24. Then, lactic acid, low-molecular-weight lactic acid oligomers, and lactide are removed from the gas. The resultant is refluxed into the upper distillation column 13.

The vapor that has not been condensed in the condenser 24 enters the subsequent condenser 25 and then condensed or liquefied therein. The liquefied impurities were generally discarded in most cases. The gas that has not been condensed in the subsequent condenser 25 is discharged outside the system via the vacuum pump 26.

Lactide discharged from the lactide purification device 15 is sent to the ring-opening polymerization device 17. In the ring-opening polymerization device 17, ring-opening polymerization reaction of lactide is allowed to proceed. The reaction is carried out by allowing lactide to come into contact with a ring-opening polymerization catalyst and a polymerization initiator under vacuum pressure of 10 torr or less and at a temperature of 120°C to 250°C.

In the catalyst regeneration device 27, a depolymerization reaction is allowed to proceed in order to reduce the molecular weight of the residue discharged from the depolymerization device 11. A reaction is carried out under vacuum pressure of 10 torr or less and at a temperature of 120°C to 250°C. Since the residue already contains a depolymerization catalyst, a depolymerization reaction proceeds when placed in the above environment. Lactide in the form of a gas generated in the reaction is sent to the distillation column 28 and the residue containing the catalyst is refluxed to the depolymerization device 11. However, if the residue contains many impurities and thus is regarded as not contributing to a depolymerization reaction, the residue may be discarded.

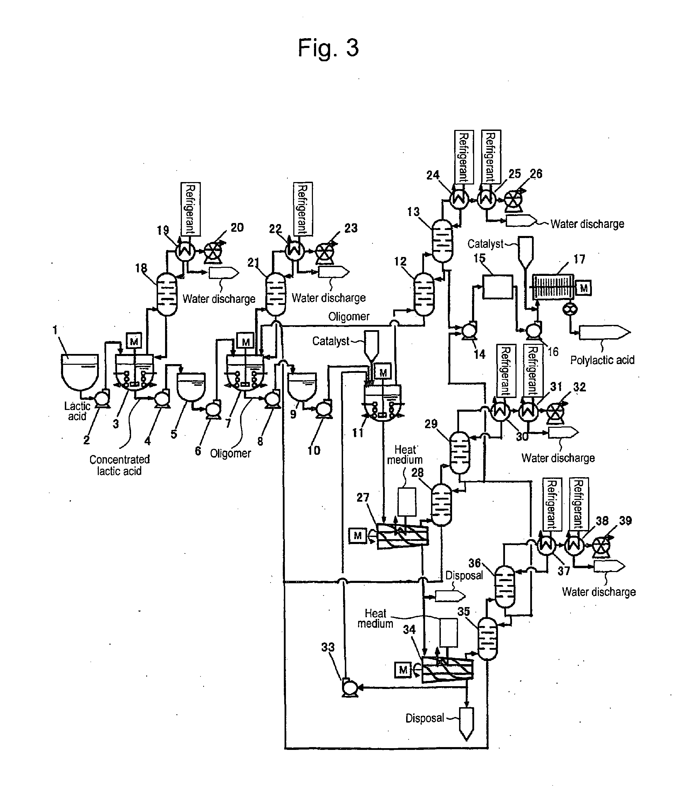

Next, the embodiment shown in fig. 3 is described below.

In the lactic acid concentration device 3, the moisture contained in lactic acid is evaporated by heating. Heating is carried out in the presence of a flow of nitrogen gas at 120°C to 150°C.

In the lactic acid concentration reaction, moisture and lactic acid are formed into a gas. The gas enters the distillation column 18. Then, lactic acid is removed from the gas and refluxed into the lactic acid concentration device 3.

Concentrated lactic acid produced in the lactic acid concentration device 3 is sent to the lactic acid condensation device 7 via the concentrated lactic acid buffer tank 5.

In the lactic acid condensation device 7, a lactic acid condensation reaction proceeds. The moisture formed as a result of the reaction is evaporated. The reaction is carried out under vacuum pressure of 10 torr or less and at a temperature of 120°C to 250°C. In the lactic acid condensation reaction, the moisture, lactic acid, low-molecular-weight lactic acid oligomers, and lactide generated as a result of decomposition of lactic acid oligomers are formed into a gas. The gas is transferred from the lactic acid condensation device 7 to the vacuum pump 23. The gas enters the distillation column 21. Lactic acid, low-molecular-weight lactic acid oligomers, and lactide are removed from the gas and refluxed into the lactic acid condensation device 7.

Oligomers generated in the lactic acid condensation device 7 are sent to the depolymerization device 11.

In the depolymerization device 11, an oligomer depolymerization reaction is allowed to proceed. The reaction is carried out by allowing lactic acid oligomers to come into contact with a depolymerization catalyst under vacuum pressure of 10 torr or less and at a temperature of 120°C to 250°C. Lactide in the form of a gas generated as a result of the reaction is sent to the distillation column 12.

In the distillation column 12, lactide in the form of a gas is cooled such that impurities contained in lactide, such as lactic acid oligomers, are liquefied. Lactide in the form of a gas is sent to the upper distillation column 13. Most of lactic acid oligomers separated from lactide have smaller molecular weights as a result of a depolymerization reaction. In order to improve the lactide yield, it is desirable to recondense low-molecular-weight oligomers for use. Therefore, the low-molecular-weight oligomers separated from lactide are refluxed into a lactic acid condensation device 7.

Lactide in the form of a gas is cooled and condensed in the upper distillation column 13 and then sent to the lactide purification device 15. The gas separated from lactide having a high water vapor content enters the condenser 24. Then, lactic acid, low-molecular-weight lactic acid oligomers, and lactide are removed from the gas. The resultant is refluxed into the upper distillation column 13.

The vapor that has not been condensed in the condenser 24 enters the subsequent condenser 25 and then condensed or liquefied therein. The liquefied impurities were generally discarded in most cases. The gas that has not been condensed in the subsequent condenser 25 is discharged outside the system via the vacuum pump 26.

Lactide discharged from the lactide purification device 15 is sent to the ring-opening polymerization device 17. In the ring-opening polymerization device 17, ring-opening polymerization reaction of lactide is allowed to proceed. The reaction is carried out by allowing lactide to come into contact with a ring-opening polymerization catalyst and a polymerization initiator under vacuum pressure of 10 torr or less and at a temperature of 120°C to 250°C.

In the catalyst regeneration device 27, a depolymerization reaction is allowed to proceed in order to reduce the molecular weight of the residue discharged from the depolymerization device 11. A reaction is carried out under vacuum pressure of 10 torr or less and at a temperature of 120°C to 250°C. Since the residue already contains a depolymerization catalyst, a depolymerization reaction proceeds when placed in the above environment. Lactide in the form of a gas generated in the reaction is sent to the distillation column 28 and the residue containing the catalyst is refluxed to the catalyst regeneration device 34. However, if the residue contains many impurities and thus is regarded as not contributing to a depolymerization reaction, the residue may be discarded.

In the catalyst regeneration device 34, a depolymerization reaction is allowed to proceed in order to reduce the molecular weight of the residue discharged from the catalyst regeneration device 27. A reaction is carried out under vacuum pressure of 10 torr or less and at a temperature of 120°C to 250°C. Since the residue already contains a depolymerization catalyst, a depolymerization reaction proceeds when placed in the above environment. Lactide in the form of a gas generated in the reaction is sent to the distillation column 35 and the residue containing the catalyst is refluxed to the depolymerization device 11 or the catalyst regeneration device 27. However, if the residue contains many impurities and thus is regarded as not contributing to a depolymerization reaction, the residue may be discarded.

### Examples

### [Example 1]

Polylactic acid was produced with the use of a polylactic acid production apparatus shown in fig. 1. Lactic acid oligomers with a number-average molecular weight of 630 were introduced as starting material to a depolymerization device 11. A reaction in the depolymerization device 11 was carried out under vacuum pressure of 10 torr or less and at a temperature of 200°C. The retention time for lactic acid oligomers in the depolymerization device 11 was 5 hours, the liquid film thickness (liquid depth) was 5 cm, and the concentration of a catalyst (tin 2-ethylhexanoate) was 0.7 kg/m³.

A reaction in the catalyst regeneration device 27 was carried out under vacuum pressure of 10 torr or less and at a temperature of 200°C.

The rate of optical isomerization in the depolymerization step increased by 0.9% (determined immediately before the lactide purification device 15).

Herein, the oligomer retention time was defined by the molten oligomer supply flow / the molten oligomer retention amount in the oligomer depolymerization device 11, which is obtained when the molten oligomer supply flow equals to the flow of condensate of vapor discharged from the depolymerization device and the liquid film thickness is stabilized.

In addition, regarding polylactic acid obtained by subjecting the obtained lactide to a ring-opening polymerization reaction, the b value representing the quality of polylactic acid in terms of coloring was 2.5. Therefore, it was thought that the influence of thermal decomposition of lactide and oligomers upon depolymerization was small. A reaction in the ring-opening polymerization device 17 was carried out under vacuum pressure of 10 torr or less and at a temperature of 200°C. The concentration of a polymerization initiator upon ring-opening polymerization was 700 ppm. The weight-average molecular weight of the obtained polylactic acid was approximately 200000.

Based on the above, it is understood that a depolymerization reaction efficiently proceeds in the present invention and thus high-purity lactide can be obtained without a decrease in the optical purity of lactide. In addition, the viscosity of the residue that has been conventionally discarded is reduced and the catalyst activity is regenerated such that the residue can be refluxed into the depolymerization device and then lactide is produced from the residue. Accordingly, the improvement of the yield and safety operation of the plant can be realized. In addition, it is understood that high quality polylactic acid can be obtained as a result of the above.

### [Example 2]

Polylactic acid was produced with the use of a polylactic acid production apparatus shown in fig. 3. Lactic acid oligomers with a number-average molecular weight of 630 were introduced as starting material to a depolymerization device 11. A reaction in the depolymerization device 11 was carried out under vacuum pressure of 10 torr or less and at a temperature of 200°C. The retention time for lactic acid oligomers in the depolymerization device 11 was designate as 5 hours, the liquid film thickness (liquid depth) was 5 cm, and the concentration of a catalyst (tin 2-ethylhexanoate) was 0.7 kg/m³

A reaction in the catalyst regeneration device 27 was carried out under vacuum pressure of 10 torr or less and at a temperature of 200°C.

The rate of optical isomerization in the depolymerization step increased by 0.9% (determined immediately before the lactide purification device 15).

Herein, the oligomer retention time was defined by the molten oligomer supply flow / the molten oligomer retention amount in the oligomer depolymerization device 11, which is obtained when the molten oligomer supply flow equals to the flow of condensate of vapor discharged from the depolymerization device and the liquid film thickness is stabilized.

In addition, regarding polylactic acid obtained by subjecting the obtained lactide to a ring-opening polymerization reaction, the b value representing the quality of polylactic acid in terms of coloring was 2.5. Therefore, it was thought that the influence of thermal decomposition of lactide and oligomers upon depolymerization was small. A reaction in the ring-opening polymerization device 17 was carried out under vacuum pressure of 10 torr or less and at a temperature of 200°C. The concentration of a polymerization initiator upon ring-opening polymerization was 700 ppm. The weight-average molecular weight of the obtained polylactic acid was approximately 200000.

Based on the above, it is understood that a depolymerization reaction efficiently proceeds in the present invention and thus high-purity lactide can be obtained without a decrease in the optical purity of lactide. In addition, the viscosity of the residue that has been conventionally discarded is reduced and the catalyst activity is regenerated such that the residue can be refluxed into the depolymerization device and then lactide is produced from the residue. Accordingly, the improvement of the yield and safety operation of the plant can be realized. In addition, it is understood that high quality polylactic acid can be obtained as a result of the above.

### [Comparative Example]

Batch-type depolymerization was carried out by using a conventional apparatus having no catalyst regeneration device with the use of lactic acid oligomers having a number-average molecular weight of 630 as the same in Examples 1 and 2. A depolymerization reaction was carried out under conditions of a depolymerization hour of 10 hours, an initial liquid face height of 80 cm, and an initial catalyst concentration of 5 kg/m³. The depolymerization reaction was carried out under vacuum pressure of 10 torr or less and at a temperature of 200°C. As a result, regarding generated lactide, the rate of optical isomerization in the depolymerization step increased by 9%.

Regarding polylactic acid obtained by subjecting the obtained lactide to a ring-opening polymerization reaction, the b value representing the quality of polylactic acid in terms of coloring was 4.4. A reaction in the ring-opening polymerization device was carried out under vacuum pressure of 10 torr or less and at a temperature of 200°C. The concentration of a polymerization initiator upon ring-opening polymerization was 700 ppm.

Based on the above results, it is understood that a depolymerization reaction does not efficiently proceed in the embodiment in which the catalyst regeneration device is not used. Further, it is understood that a reaction of isomerization of lactic acid oligomers tends to take place and impurities are generated as a result of thermal decomposition of lactic acid oligomers, leading to a decrease in the quality of polylactic acid.

All publications, patents, and patent applications cited herein are incorporated herein by reference in their entirety.

### Industrial Applicability

According to the present invention, the viscosity of a residue discharged from a depolymerization device is reduced with the use of a catalyst regeneration device so as to restore the catalyst activity and recover lactide from the residue. In addition, the resultant can be refluxed into a depolymerization device in some cases. Therefore, polylactic acid can be produced at a high starting material yield in a stable manner.

## Claims

1. A method for continuously or intermittently producing lactide by depolymerizing lactic acid oligomers, comprising the steps of:
a) subjecting lactic acid oligomers to a depolymerization reaction for synthesizing lactide in a depolymerization device;
b) subjecting a residue in the depolymerization device to a depolymerization reaction in a reaction vessel having an evaporation area per unit volume of solution larger than that of the reaction vessel of the depolymerization device, thereby reducing the molecular weights of lactic acid oligomers contained in the residue; and
c) recovering lactide generated in a gas form as a result of the depolymerization reaction via condensation.

2. The method according to claim 1, further comprising a step of feeding the lactic acid oligomers with reduced molecular weights that have been generated in step "b" back to the depolymerization device.

3. The method according to claim 1, wherein the residue in a molten form is formed into a thin layer with external force in step "b."

4. The method according to claim 2, wherein the residue in a molten form is formed into a thin layer with external force in step "b."

5. The method according to any one of claims 1 to 4, wherein the depolymerization reaction is carried out with the use of a centrifugal thin film evaporator in step "b."

6. The method according to any one of claims 1 to 4, further comprising a step of synthesizing lactic acid oligomers via condensation of lactic acid.

7. A method for producing polylactic acid, comprising producing lactide by depolymerizing lactic acid oligomers by the method according to any one of claims 1 to 4 and subjecting the obtained lactide to ring-opening polymerization.

8. A method for producing polylactic acid, comprising producing lactide by depolymerizing lactic acid oligomers by the method according to claim 5 and subjecting the obtained lactide to ring-opening polymerization.

9. A method for producing polylactic acid, comprising producing lactide by depolymerizing lactic acid oligomers by the method according to claim 6 and subjecting the obtained lactide to ring-opening polymerization.

10. A lactide production apparatus for continuously or intermittently producing lactide, comprising:
a depolymerization device for subjecting lactic acid oligomers to a depolymerization reaction;
a catalyst regeneration device, which has a reaction vessel having an evaporation area per unit volume of solution larger than that of the reaction vessel of the depolymerization device and is for subjecting a residue in the depolymerization device to a depolymerization reaction in order to reduce the molecular weights of lactic acid oligomers contained in the residue; and
a distillation column for condensing lactide generated in a gas form as a result of the depolymerization reaction.

11. The apparatus according to claim 10, further comprising a liquid-feeding pump for feeding lactic acid oligomers with decreased molecular weights generated in the catalyst regeneration device to the depolymerization device.

12. The apparatus according to claim 10, wherein the catalyst regeneration device has a means for forming a thin layer of the residue in a molten form.

13. The apparatus according to claim 11, wherein the catalyst regeneration device has a means for forming a thin layer of the residue in a molten form.

14. The apparatus according to any one of claims 10 to 13, wherein the catalyst regeneration device is a centrifugal thin film evaporator.

15. The apparatus according to any one of claims 10 to 13, further comprising a lactic acid condensation device for producing lactic acid oligomers via condensation of lactic acid.

16. A polylactic acid production apparatus for continuously or intermittently producing polylactic acid, comprising:
the lactide production apparatus according to any one of claims 10 to 13; and
a ring-opening polymerization device for ring-opening polymerization of lactide.

17. A polylactic acid production apparatus for continuously or intermittently producing polylactic acid, comprising:
the lactide production apparatus according to claim 14; and
a ring-opening polymerization device for ring-opening polymerization of lactide.

18. An polylactic acid production apparatus for continuously or intermittently producing polylactic acid, comprising:
the lactide production apparatus according to claim 15; and
a ring-opening polymerization device for ring-opening polymerization of lactide.

19. A method for continuously or intermittently producing lactide by depolymerizing lactic acid oligomers, comprising the steps of:
a) subjecting lactic acid oligomers to a depolymerization reaction for synthesizing lactide in a depolymerization device;
b) subjecting a residue in the depolymerization device to a depolymerization reaction in a reaction vessel having an evaporation area per unit volume of solution larger than that of the reaction vessel of the depolymerization device and synthesizing lactide from lactic acid oligomer contained in the residue; and
c) recovering lactide generated in a gas form as a result of the depolymerization reaction via condensation.
